# EUROPÄISCHE PATENTSCHRIFT

(11) **EP 3 755 393 B1**
(45) Veröffentlichungstag und Bekanntmachung des Hinweises auf die Patenterteilung: **26.04.2023**
(21) Anmeldenummer: 19705495.0
(22) Anmeldetag: 14.02.2019
(51) Int. Cl.: A61M 1/16, A61M 1/28, A61J 1/10, A61J 1/14

(54) **MAGAZIN MIT LÖSUNGSBEUTELN FÜR DIE DIALYSE UND VERFAHREN ZU DEREN BEFÜLLUNG**
MAGAZINE WITH DIALYSIS SOLUTION BAGS AND METHOD FOR FILLING THEM
MAGASIN AVEC POUCHES DE SOLUTION DE DIALYSE ET PROCÉDÉ DE REMPLISSAGE DES POUCHES

(30) Priorität: 21.02.2018 DE 102018103950
(43) Veröffentlichungstag der Anmeldung: 30.12.2020
(73) Patentinhaber: Fresenius Medical Care Deutschland GmbH, 61352 Bad Homburg (DE)
(72) Erfinder: WABEL, Peter, 61191 Rosbach (DE); BERLICH, Robert, 66606 St. Wendel (DE); BREUNINGER, Marcus, 61352 Bad Homburg (DE); STAUDE, Birgit, 64319 Pfungstadt (DE); WEISS, Stefan, 61352 Bad Homburg (DE); CERMAN, Zdenek, 65126 Idstein (DE); RAU, Matthias, 65195 Wiesbaden (DE)
(74) Vertreter: Herrmann, Uwe
(86) Internationale Anmeldenummer: PCT/EP2019/053667
(87) Internationale Veröffentlichungsnummer: WO 2019/162183

(56) Entgegenhaltungen:
- JP-A- H06 285 136
- US-A1- 2009 009 179

## Beschreibung

Die Erfindung betrifft ein Magazin mit Lösungsbeuteln für die Dialyse sowie ein Verfahren zu deren Befüllung.

Insbesondere im Bereich der Peritonealdialyse ist es üblich, dem Patienten Lösungsbeutel zur Verfügung zu stellen, die mit einer für den Patienten geeigneten Dialyselösung befüllt sind. Diese Lösungsbeutel schließt der Patient dann selbsttätig oder mit Unterstützung von medizinischem Fachpersonal an einen Einlaufschlauch an, um das Peritoneum mit der Lösung zu füllen.

In der Herstellung werden vorgefertigte Beutel durch einen Befüllschlauch oder durch eine sonstige Befüllöffnung mit Dialyselösung bzw. Lösungskonzentrat und Wasser befüllt und der Befüllschlauch bzw. die Öffnung sodann versiegelt. Die Herstellung und Befüllung der Beutel erfolgte bisher regelmäßig im Werk des Herstellers. In der jüngeren Vergangenheit wurden jedoch Konzepte zur dezentralen Befüllung der Dialysebeutel vorgestellt, wobei in diesem Zusammenhang beispielsweise auf die WO 2017/127632 A1 verwiesen werden kann.

Aus der US 2009/009179 A1 ist zudem ein Prüfsystem für ein offenes Siegel für einen Mehrkammer-Vorratsbehälter mit mindestens einem länglichen Siegel bekannt.

Aufgabe der Erfindung ist es, ein Konzept zur Bereitstellung und Befüllung von Beuteln mit Dialyselösung vorzuschlagen, das Insbesondere im Rahmen einer dezentrale Beutelbefüllung zu einer Vereinfachung und erhöhten Prozesssicherheit führen kann.

Vor diesem Hintergrund betrifft die Erfindung ein Magazin mit mehreren darin auf genommenen Lösungsbeuteln für die Dialyse, wobei die mehreren Lösungsbeutel Identisch sind, wobei die mehreren Lösungsbeutel an unterschiedlichen aber identisch ausgestalteten Halteplätzen des Magazins in identischer Ausrichtung ortsfest fixiert sind und wobei die Halteplätze so ausgebildet sind, dass die Lösungsbeutel aus dem Magazin entnommen werden können.

Ein derartiges Magazin kann insgesamt zu einer dezentralen Befüllstation transportiert und dort als Verbrauchsmaterial in einen Befüllroboter eingesetzt werden. Dort können einzelne Lösungsbeutel in automatisierter Weise nach und nach aus dem Magazin entnommen und sodann befüllt werden. Durch die identische Ausgestaltung der Halteplätze und Positionierung der Lösungsbeutel an den Halteplätzen wird die Automatisierung des Vorgangs ermöglicht bzw. vereinfacht.

In einer Ausführungsform ist vorgesehen, dass es sich bei den Lösungsbeuteln um Beutel aus verbundenen und vorzugsweise verschweißten Kunststofffolien handelt. Die Folien können transparent ausgebildet sein, um die Füllung optisch erkennen zu können. Femer können diese mehrschichtig ausgeführt, um eine unter anderem für bicarbonatgepufferte Lösungen erforderliche hohe Barrierewlrkung für Gase erreichen zu können.

In einer Ausführungsform Ist vorgesehen, dass die Lösungsbeutel einen nicht Im direkten Kontakt mit dem Innenvolumen stehenden Materialüberstand aufweisen, in dem vorzugsweise wenigstens ein Loch oder eine materialgeschwächte Zone eingearbeitet ist, wobei es sich bei dem Materialüberstand vorzugsweise um eine umlaufende Schweißnaht handelt

In einer Ausführungsform ist vorgesehen, dass die Lösungsbeutel zumindest in ihren vier Eckbereichen jeweils ein Loch oder eine materialgeschwächte Zone aufweisen. Auch eine größere Anzahl an über den Umfang verteilten Löcher oder materialgeschwächten Zonen Ist aber In einer alternativen Variante denkbar. Die Löcher oder materialgeschwächten Zonen sind vorzugsweise im Wesentlichen punktförmig. Sie können dazu dienen, um einen Stift durch die Lösungsbeutel führen zu können und so eine ortsfeste Fixierung im Magazin zu erlauben.

In einer Ausführungsform Ist vorgesehen, dass jeder Lösungsbeutel einen Befüllanschluss und/oder einen Entnahmeanschluss aufweist. Die Anschlüsse können durch Leitungen realisiert sein, die zwischen den Folienlagen des Lösungsbeutels eingeschlossen sind und von außen in das Innenvolumen des Lösungsbeutels reichen. Vorzugsweise sind die Leitungen von einer Schweißnaht des Beutels umschlossen. Die Entnahmeanschlüsse und Befüllanschlüsse eines Beutels können jeweils nebeneinander an derselben Kante des Beutels angeordnet sein. Derartige Anschlüsse sind besonders einfach und mit geringem Materialaufwand zu produzieren und führen zu einer flachen Beutelgestalt.

In einer Ausführungsform ist vorgesehen, dass das Magazin eine Mehrzahl an identischen Trägem aufweist, in denen je ein Lösungsbeutel fixiert ist, wobei die Träger direkt aneinander gestapelt sind. Die Stapelung kann in horizontaler Richtung im Sinne einer Stapelung nebeneinander und in vertikaler Richtung im Sinne einer Stapelung übereinander erfolgen. Bevorzugt Ist eine Stapelung in vertikaler Richtung. Die Träger können beispielsweise aus einem starren Kunststoffmaterial gefertigt sein. Die gegenüberliegenden Seiten der Träger können gegebenenfalls mit korrespondierenden Positionierhilfen versehen sein, um die relative Lage zueinander zu fixieren. Geeignete Positionierhilfen umfassen korrespondierende Stege und Rastpositionen.

In einer Ausführungsform ist vorgesehen, dass die Träger ein flächiges Basiselement und eine sich in Normalrichtung erstreckende Umrahmung aufweisen, wobei der Lösungsbeutel auf dem Basiselement aufliegt und von der Umrahmung umgeben wird. Diese Träger können im Magazin so aneinander gestapelt sein, dass die Basiselemente der Träger mit ihrer Unterseite auf den Umrahmungen der jeweils vorhergehenden Träger aufliegen und so zwischen den Basiselementen benachbarter Träger und den Umrahmungen Volumina eingeschlossen werden, in denen sich die Lösungsbeutel befinden. Die Höhe der Umrahmung kann vorzugsweise so gewählt sein, dass eine Ausbauchung der Lösungsbeutel nur in einem sehr geringen Umfang ermöglicht wird. So kann in einem Fall, in dem die Lösungsbeutel mit einem flüssigen Konzentrat vorbefüllt sind, eine gleichmäßige Verteilung des Konzentrats über die Innenvolumina der Lösungsbeutel sichergestellt werden.

In einer Ausführungsform ist vorgesehen, dass die Träger wenigstens einen und vorzugsweise wenigstens vier vom Basiselement in Normalrichtung abstehende Stifte aufweisen, wobei vorzugsweise vorgesehen ist, dass die Stifte durch Materialabschnitte des Lösungsbeutels geführt sind, um diesen ortsfest zu fixieren. Mehrere Stifte können so angeordnet sein, dass sie In regelmäßigen Abständen über den Umfang des Lösungsbeutels verteilt angeordnet und im Randbereich geführt sind, um diesen in einer ausgebreiteten Lage fixieren zu können. Im Falle von vier Stiften können diese und der Lösungsbeutel so angeordnet sein, dass die Stifte durch die Ecken des Lösungsbeutels geführt sind und diesen so in einer ausgebreiteten Lage fixieren können. Sofern die Lösungsbeutel Löcher oder materialgeschwächte Zonen aufweisen, beispielsweise in einer umlaufenden Schweißnaht, können die Stifte durch die Löcher oder materialgeschwächte Zonen geführt sein. Andernfalls ist auch ein Durchstechen der Beutelfolie mit gespitzten Stiften denkbar.

Alternativ oder zusätzlich zu den Stiften können die Träger beispielsweise Klemmen oder Saugnäpfe aufweisen, um den Lösungsbeutel ortsfest und in ausgebreiteter Lage darin fixieren zu können.

In einer Ausführungsform ist vorgesehen, dass das Magazin einen gemeinsamen Rahmen mit mehreren Steckplätzen aufweist, an welche die mehreren Lösungsbeutel gesteckt sind. Eine solche Ausgestaltung kann den Vorteil haben, dass das Magazin weniger einzelne Teile umfasst.

In einer Ausführungsform ist vorgesehen, dass der Rahmen eine Lochplatte mit mehreren Langlöchern und die Lösungsbeutel jeweils einen Zapfen aufweisen, wobei die Langlöcher jeweils einen vorzugsweise durch Haltevorsprünge abgetrennten Arretierungsplatz an einem Ende des Langlochs aufweisen sowie am anderen Ende eine Erweiterung aufweisen oder zum Rand der Lochplatte hin offen sind, und wobei die Zapfen einen Kragen aufweisen, dessen Durchmesser größer als die Breite des Langlochs aber kleiner als der Durchmesser der Erweiterung Ist. Die Zapfen können beispielsweise durch aus dem Lösungsbeutel hervorstehende Abschnitte eines Befüll- oder Entnahmeanschlusses gebildet werden. Die Lösungsbeutel können mit ihren Zapfen in die Arretierungsplätze der Langlöcher eingesetzt sein, wobei der Kragen gegen eine Bewegung normal zur Lochplatte sichert. Die Lösungsbeutel können nur so aus den Arretierungsplätzen entnommen werden, Indem die Zapfen in der Ebene der Lochplatte in den Langlöchem verschoben werden und, gegen einen etwaigen Widerstand beim Überwinden der Haltevorsprünge, von den Arretierungsplätzen zur Erweiterung oder zum Rand der Lochplatte bewegt werden. Diese Bewegung kann in einem Befüllroboter in automatisierter Weise vollzogen werden. Die Lochplatte ist vorzugsweise horizontal ausgerichtet und die Lösungsbeutel erstrecken sich vorzugsweise ausgehend vom Zapfen nach unten.

In einer Ausführungsform ist vorgesehen, dass der Rahmen lösbar an einer Box befestigt ist und vorzugsweise an den Oberkanten gegenüberliegender Seitenwände der Box eingehängt ist. Zu diesem Zweck kann der Rahmen an gegenüberliegenden Seiten eine Einhängevorrichtung aufweisen. Sofern die Lösungsbeutel sich ausgehend vom Rahmen nach unten erstrecken, können diese so innerhalb der Box aufgenommen und geschützt sein. Der Rahmen kann beispielsweise zum Transport an der Box befestigt sein und in einer Befüllstation manuell oder automatisch aus der Box entnommen werden. Er kann aber auch in der Befüllstation an der Box befestigt bleiben.

In einer Ausführungsform Ist vorgesehen, dass die Lösungsbeutel mit einem Konzentrat zur Herstellung einer Dialyselösung befüllt sind. Die Lösungsbeutel des Magazins können dabei alle mit demselben Konzentrat oder mit unterschiedlichen Konzentraten befüllt sein. Das Konzentrat kann weniger als 40% und vorzugsweise weniger als 20% des Fassungsvolumens des Beutels einnehmen. Die so vorbefüllten Beutel benötigen wesentlich weniger Platz als Beutel, die bereits mit einer fertigen Dialyselösung befüllt sind.

Die Erfindung betriff ferner ein Verfahren zur Befüllung der Lösungsbeutel eines Magazins nach einem der vorhergehenden Ansprüche, wobei das Magazin in einen Befüllroboter eingesetzt wird und sodann einzelne Lösungsbeutel im Rahmen eines vorzugsweise automatisierten Vorgangs entweder aus dem Magazin entnommen und sodann befüllt oder befüllt und sodann aus dem Magazin entnommen werden. Nach der Befüllung kann der Befüllanschluss versiegelt werden. Die Befüllung der Lösungsbeutel durch den Befüllroboter kann Im Falle von mit einem flüssigen oder festen Lösungskonzentrat vorbefüllten Lösungsbeuteln mit Wasser oder andernfalls mit einer Dialyselösung erfolgen.

Das Verfahren kann vorzugsweise in einer dezentralen und gegebenenfalls mobilen Einheit zur Herstellung von Dialyselösungen vor Ort und zur direkten Abgabe an den Patienten durchgeführt werden. Die Vorbefüllung der Lösungsbeutel mit Konzentrat, sofern vorhanden, kann in diesem Fall bereits im Werk vorgenommen werden, sodass das Magazin schon im vorbefüllten Zustand an eine dezentrale Befülleinheit transportiert werden können.

Weiterhin betrifft die Erfindung ein System aus einem Befüllroboter und einem Magazin nach einem der Ansprüche 1 bis 11, wobei der Befüllroboter eine Aufnahme umfasst, in welcher das Magazin passgenau aufgenommen werden kann. Der Befüllroboter kann eine Steuereinheit umfassen, die ausgebildet ist, ein erfindungsgemäßes Verfahren durchzuführen. Der Befüllroboter kann in einer wie oben beschriebenen dezentralen Einheit zur zur Herstellung von Dialyselösungen vor Ort und zur direkten Abgabe an den Patienten angeordnet sein.

In einer Ausführungsform Ist vorgesehen, dass das Magazin automatisch in eine Aufnahme des Befüllroboters eingebracht wird, sodass die Lösungsbeutel in einer zur Entnahme und ggf. Befüllung geeigneten Position vorliegen, und das Magazin nach der Entnahme automatisch wieder aus der Aufnahme entfernt wird. Beispielsweise kann ein Einzug oder ein Einheben von einer Ladefläche einer dezentralen Einheit vorgesehen sein. Die Aufnahme des Befüllroboters kann so ausgebildet sein, dass das Magazin passgenau aufgenommen wird.

Weitere Einzelheiten und Vorteile der Erfindung ergeben sich aus den nachfolgend anhand der Figuren beschriebenen Ausführungsbeispielen. In den Figuren zeigen:
- Figur 1:: eine Ausführungsform eines erfindungsgemäßen Magazins;
- Figur 2:: mögliche Varianten der Fixierung eines Lösungsbeutels in Trägem des Magazins gemäß Figur 1; und
- Figur 3:: eine weitere Ausführungsform eines erfindungsgemäßen Magazins.

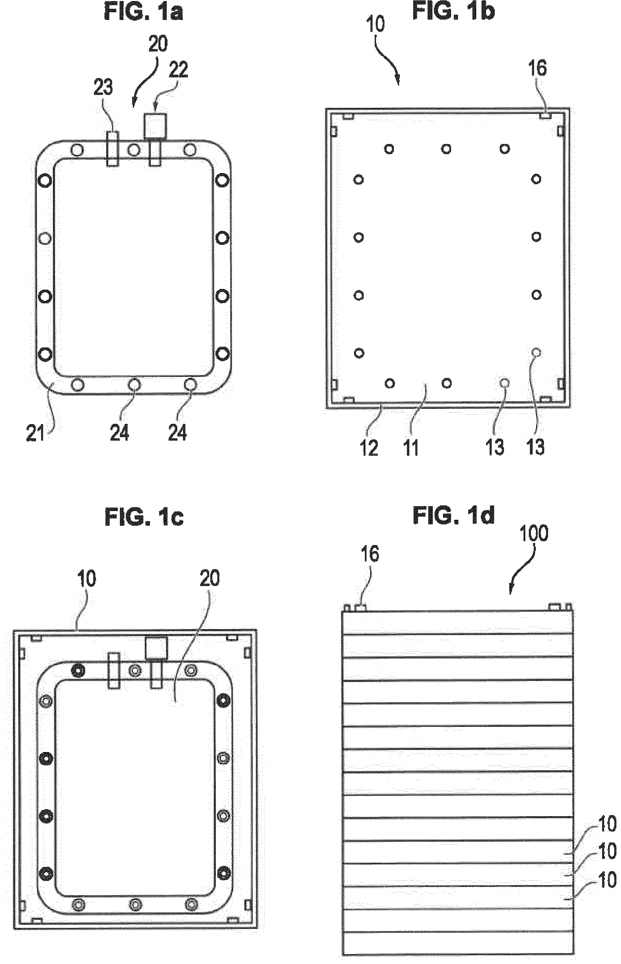

In Figur 1 ist eine Ausführungsform eines erfindungsgemäßen Magazins 100 gezeigt. Das Magazin 100, wie gezeigt In Figur 1d, besteht aus einer Vielzahl an identischen Trägern 10, dargestellt In Figur 1c, in denen je ein Lösungsbeutel 20 fixiert ist. Ein einzelner Lösungsbeutel 20 ist In Figur 1a gezeigt, ein Träger 10 mit darin fixiertem Lösungsbeutel 20 in Figur 1c. Die Träger 10 weisen jeweils eine flache rechteckige Bodenplatte 11 auf, die von einer sich in Normalrichtung nach oben erstreckenden Umrahmung 12 umgeben ist. Sie sind aus einem starren Kunststoffmaterial gefertigt.

Die ebenfalls im Wesentlichen rechteckigen Lösungsbeutel 20, deren Abmessungen aber kleiner als die Abmessungen der Bodenplatten 11 sind, liegen auf den Bodenplatten 11 auf. Sie werden von der Umrahmung 12 umgeben, berühren diese aber nicht. Die Lösungsbeutel 20 sind durch umfängliches Verschweißen zweier Kunststofffolien gebildet und weisen entsprechend eine umlaufende Schweißnaht 21 auf. Die Kunststofffolien sind mehrschichtig ausgeführt und transparent. Die Lösungsbeutel 20 weisen jeweils einen Befüllanschluss 22 und einen Entnahmeanschluss 23 auf. Die Anschlüsse 22 und 23 sind durch von außen In das Innenvolumen des Lösungsbeutels 20 reichende Leitungen realisiert, die von einer Schweißnaht 21 des Beutels 20 umschlossen und mithin zwischen den Folienlagen des Lösungsbeutels 20 eingeschlossen sind. Die Anschlüsse 22 und 23 sind nebeneinander an derselben Kante des Beutels 20 angeordnet.

Um die Beutel 20 ortsfest in einer ausgebreiteten Lage auf den Bodenplatten 11 fixieren zu können, sind in der Schweißnaht 21 der Beutel 20 In regelmäßigen Abständen über den Beutelumfang verteilt Löcher 24 eingearbeitet. Die Bodenplatten 11 weisen an korrespondierenden Stellen in Normalrichtung nach oben stehende Stifte 13 auf, die durch die Löcher 24 des Lösungsbeutels 20 geführt sind.

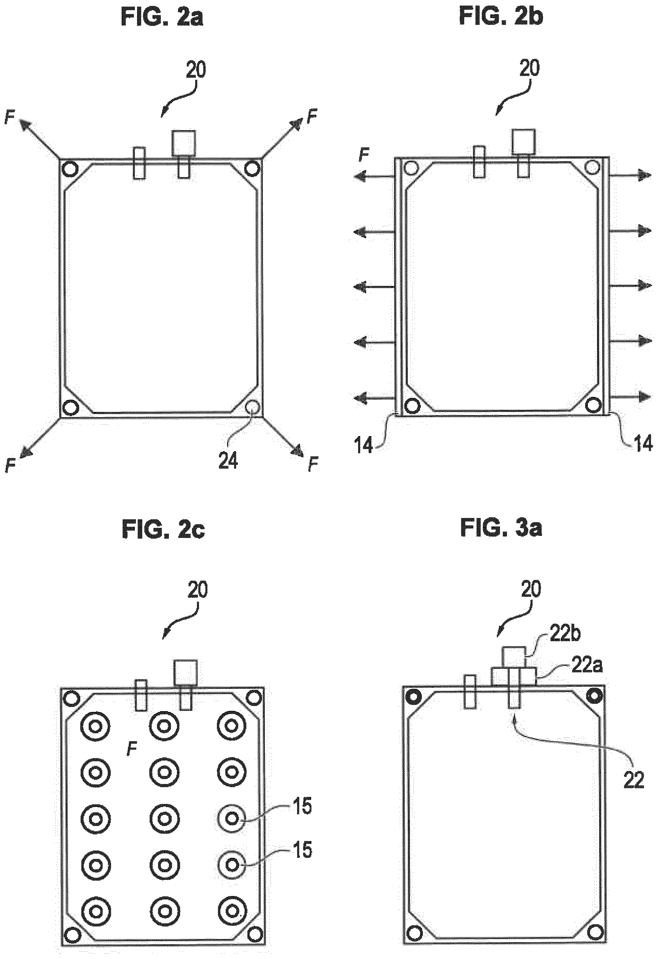

Alternative Varianten einer Fixierung der Beutel 20 auf den Bodenplatten 11 sind in Figur 2 gezeigt, in einer Variante gemäß Figur 2a sind lediglich vier Löcher 24 an den vier Eckpunkten des Beutels 20 vorhanden, durch die korrespondierende Stifte 13 der Träger 10 geführt sind. Wie in der Figur 2a durch Pfeile angedeutet ist, genügt die Fixierung an diesen vier Punkten, um den Beutel 20 in einer ausgestreckten Lage festzusetzen. In einer weiteren Variante gemäß Figur 2b ist der Beutel 20 an zwei gegenüberliegenden Seiten jeweils über die gesamte Länge dadurch gehalten, dass die Schweißnaht 21 in einer Klemme 14 fixiert ist. In einer wiederum anderen Variante gemäß Figur 2c sind auf der Bodenplatte 11 verteilt Saugnäpfe 15 angeordnet, um den Beutel 20 zu fixieren.

Die Träger 10 werden In vertikaler Richtung direkt übereinander gestapelt, um das Magazin 100 zu bilden. Die Oberseiten und die Unterseiten der Träger 10 sind an den vier Ecken jeweils mit korrespondierenden Stegen 16 und Rastpositionen versehen, um die relative Lage der gestapelten Träger 10 zueinander zu fixieren.

Durch die Stapelung werden durch die Bodenplatten 11 benachbarter Träger 10 sowie die Umrahmungen 12 der Träger 10 Volumina eingeschlossen, in denen sich die Lösungsbeutel 20 befinden. Die Höhe der Umrahmungen 12 ist so gewählt, dass eine Ausbauchung der Lösungsbeutel 20 nur in einem sehr geringen Umfang ermöglicht wird. So kann in einem Fall, in dem die Lösungsbeutel 20 mit einem flüssigen Konzentrat vorbefüllt sind, eine gleichmäßige Verteilung des Konzentrats über die Innenvolumina der Lösungsbeutel 20 sichergestellt werden. Die Fixierung der Lösungsbeutel 20 in ihrer ausgestreckten Lage, durch die in Figur 1 gezeigte Maßnahme oder alternative die in Figur 2 dargestellten Maßnahmen, stellt ebenfalls eine gleichmäßige Verteilung des Konzentrats, sofern vorhanden, über die Innenvolumina der Lösungsbeutel 20 sicher.

Das Magazin 100 kann insgesamt zu einer dezentralen Befüllstation transportiert und dort als Verbrauchsmaterial In einen Befüllroboter eingesetzt werden. Dort können einzelne Träger 10 in automatisierter Weise nach und nach vom Magazin 100 genommen und sodann die Lösungsbeutel 20 aus dem Träger 10 entnommen und befüllt werden. Durch die identische Ausgestaltung der Träger 10 und identische Positionierung der Lösungsbeutel 20 in den Trägem 10 wird die Automatisierung des Vorgangs vereinfacht.

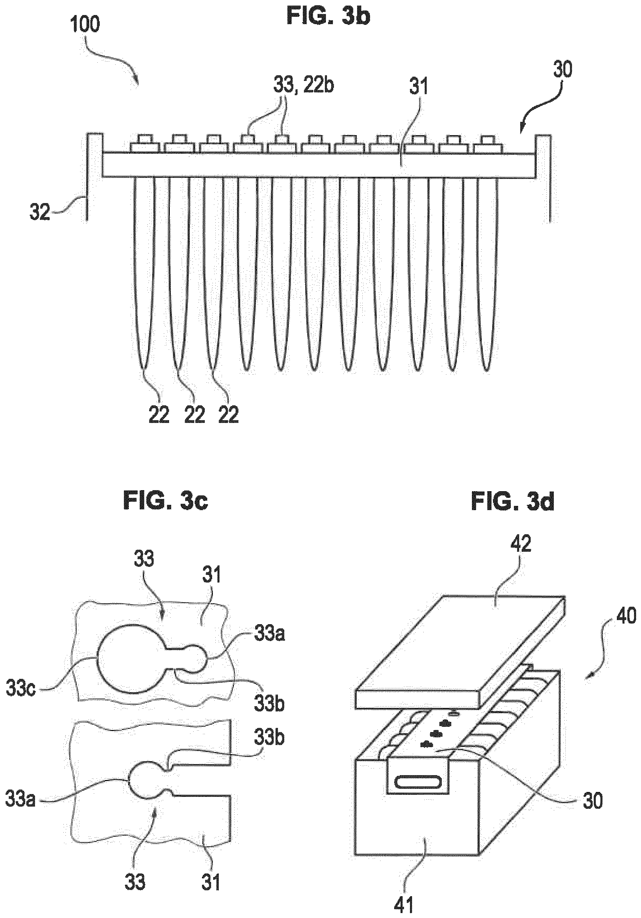

Figur 3 zeigt eine weitere Ausführungsform eines erfindungsgemäßen Magazins 100. Das Magazin 100 umfasst in dieser Ausführungsform einen gemeinsamen Rahmen 30 mit einer Vielzahl an aufgesteckten Lösungsbeuteln 20.

Die Lösungsbeutel 20 sind, wie in Figur 3a ersichtlich, im Wesentlichen identisch aufgebaut wie die in Figur 2a gezeigten Lösungsbeutel 20, mit dem einzigen Unterschied, dass der Befüllanschluss 22 nach außen hin etwas verlängert Ist und einen Halsabschnitt 22a sowie einen demgegenüber verbreiterten Kragen 22b umfasst.

Der Rahmen 30 umfasst eine längliche Lochplatte 31 sowie an gegenüberliegenden Stirnseiten der Lochplatte 31 befestigte Einhängelaschen 32, wie dies in Figur 3b zu erkennen ist. Die Lochplatte 31 umfasst mehrere Steckplätze 33 für die Lösungsbeutel 20, an denen diese mit ihrem Befüllanschluss 22 eingesteckt sind.

Die Steckplätze 33 können so ausgebildet sein, wie in den Draufsichten der Figur 3c dargestellt. Nämlich handelt es sich bei den Steckplätzen 33 um Langlöcher, die einen Arretierungsplatz 33a an einem Ende aufweisen, der durch Haltevorsprünge 33b gegenüber den anderen Zonen des Langlochs abgegrenzt wird. Am gegenüberliegenden Ende des Langlochs Ist dieses entweder erweitert (obere Abbildung der Figur 3c) oder zum Rand der Lochplatte 31 hin offen (untere Abbildung der Figur 3c). Die Befüllanschlüsse 22 der Lösungsbeutel 20 sind so in diese Steckplätze 33 eingesetzt, dass der Hals 22a das Langloch am Arretierungsplatz 33a durchläuft und der Kragen 22b die Befüllanschlüsse 22 gegen ein Lösen normal zur Lochplatte 31 sichert. Der Lösungsbeutel 20 kann nur so aus den Steckplätzen 33 entnommen werden, indem der Befüllanschluss 22 in der Ebene der Lochplatte 31 in den Langlöchern verschoben wird und gegen einen Widerstand beim Überwinden der Haltevorsprünge 33b von den Arretierungsplätzen 33a zur Erweiterung 33c (obere Abbildung der Figur 3c) oder zum Rand der Lochplatte 31 (untere Abbildung der Figur 3c) bewegt wird. Diese Bewegung kann in einem Befüllroboter in automatisierter Weise vollzogen werden.

Die Lochplatte 31 ist horizontal ausgerichtet und die Lösungsbeutel 20 erstrecken sich im eingehängten Zustand ausgehend vom Befüllanschluss 22 nach unten. Die Steckplätze 33 sind in Längsrichtung der Lochplatte 31 in Reihe gestaffelt, was entsprechend für die eingesteckten Lösungsbeutel 20 gilt.

Wie dies aus Figur 3d ersichtlich ist, ist der Rahmen 30 mit seinen Einhängelaschen 32 an den Oberkanten gegenüberliegender Seitenwände 41 einer Box 40 eingehängt. Die ausgehend von der Lochplatte 31 nach unten hängenden Beutel 20 sind so im Innenraum der Box 40 aufgenommen und geschützt. Die befüllte Box 40 kann zum Transport zusätzlich mit einem Deckel 42 abgedeckt sein.

Wie auch das Magazin 100 der Figur 1 kann auch das Magazin 100 der Figur 3 insgesamt zu einer dezentralen Befüllstation transportiert und dort als Verbrauchsmaterial in einen Befüllroboter eingesetzt werden. Während des Transports kann der Rahmen 30 an der Box 40 befestigt sein und in einer Befüllstation manuell oder automatisch aus der Box 40 entnommen und an einen Lagerplatz am Befüllroboter eingesetzt werden. Dort können einzelne Lösungsbeutel 20 in automatisierter Weise nach und nach vom Rahmen 30 genommen und sodann befüllt werden. Durch die identische Ausgestaltung aller Steckplätze 33 und die identische Positionierung der Lösungsbeutel 20 In den Steckplätzen 33 wird die Automatisierung des Vorgangs vereinfacht.

Die Lösungsbeutel 20 können bereits vor ihrem Transport zur dezentralen Befüllstation und dem dortigen Einsetzen In einen Befüllroboter beispielsweise im Werk mit Konzentrat vorbefüllt werden, sodass sie In der dezentralen Befüllstation lediglich noch mit entionisiertem Wasser, das gegebenenfalls noch variable Bestandteile der Dialyselösung aufweisen kann, die Im Konzentrat nicht berücksichtigt sind, aufgefüllt werden müssen.

## Patentansprüche

1. Magazin (100) mit mehreren darin aufgenommenen Lösungsbeuteln (20) für die Dialyse, wobei die mehreren Lösungsbeutel (20) identisch sind, **dadurch gekennzeichnet, dass** die mehreren Lösungsbeutel (20) an unterschiedlichen aber identisch ausgestalteten Halteplätzen des Magazins (100) in identischer Ausrichtung ortsfest fixiert sind und wobei die Halteplätze so ausgebildet sind, dass die Lösungsbeutel (20) aus dem Magazin (100) entnommen werden können.

2. Magazin (100) nach Anspruch 1, **dadurch gekennzeichnet, dass** es sich bei den Lösungsbeuteln (20) um Beutel aus verbundenen Kunststofffolien handelt.

3. Magazin (100) nach einem der vorhergehenden Ansprüche, **dadurch gekennzeichnet, dass** die Lösungsbeutel (20) einen nicht im direkten Kontakt mit dem Innenvolumen stehenden Materialüberstand aufweisen, wobei es sich bei dem Materialüberstand vorzugsweise um eine umlaufende Schweißnaht (21) handelt

4. Magazin (100) nach einem der vorhergehenden Ansprüche, **dadurch gekennzeichnet, dass** das Magazin (100) eine Mehrzahl an identischen Trägern (10) aufweist, in denen je ein Lösungsbeutel (20) fixiert ist, wobei die Träger (10) direkt aneinander gestapelt sind.

5. Magazin (100) nach Anspruch 4, **dadurch gekennzeichnet, dass** die Träger (10) ein flächiges Basiselement und eine sich in Normalrichtung erstreckende Umrahmung (12) aufweisen, wobei der Lösungsbeutel (20) auf dem Basiselement aufliegt und von der Umrahmung (12) umgeben wird.

6. Magazin (100) nach Anspruch 5, **dadurch gekennzeichnet, dass** die Träger (10) wenigstens einen vom Basiselement in Normalrichtung abstehende Stift (13) aufweisen.

7. Magazin (100) nach einem der Ansprüche 1 bis 3, **dadurch gekennzeichnet, dass** das Magazin (100) einen gemeinsamen Rahmen (30) mit mehreren Steckplätzen (33) aufweist, an welche die mehreren Lösungsbeutel (20) gesteckt sind.

8. Magazin (100) nach Anspruch 7, **dadurch gekennzeichnet, dass** der Rahmen (30) eine Lochplatte (31) mit mehreren Langlöchern und die Lösungsbeutel (20) jeweils einen Zapfen aufweisen, wobei die Langlöcher jeweils einen Steckplatz (33) an einem Ende des Langlochs aufweisen sowie am anderen Ende eine Erweiterung aufweisen oder zum Rand der Lochplatte hin offen sind, und wobei die Zapfen einen Kragen aufweisen, dessen Durchmesser größer als die Breite des Langlochs aber kleiner als der Durchmesser der Erweiterung ist.

9. Magazin (100) nach Anspruch 7 oder 8, **dadurch gekennzeichnet, dass** der Rahmen (30) lösbar an einer Box (40) befestigt ist.

10. Magazin (100) nach einem der vorhergehenden Ansprüche, **dadurch gekennzeichnet, dass** die Lösungsbeutel (20) mit einem Konzentrat zur Herstellung einer Dialyselösung befüllt sind.

11. Magazin (100) nach Anspruch 10, **dadurch gekennzeichnet, dass** das Konzentrat weniger als 40% des Fassungsvolumens des Beutels einnimmt.

12. Verfahren zur Befüllung der Lösungsbeutel (20) eines Magazins (100) nach einem der vorhergehenden Ansprüche, wobei das Magazin (100) in einen Befüllroboter eingesetzt wird und sodann einzelne Lösungsbeutel (20) im Rahmen eines vorzugsweise automatisierten Vorgangs entweder aus dem Magazin (100) entnommen und sodann befüllt oder befüllt und sodann aus dem Magazin (100) entnommen werden.

13. Verfahren nach Anspruch 12, **dadurch gekennzeichnet, dass** das Magazin (100) automatisch in eine Aufnahme des Befüllroboters eingebracht wird, sodass die Lösungsbeutel (20) in einer zur Entnahme und ggf. Befüllung geeigneten Position vorliegen, und das Magazin (100) nach der Entnahme automatisch wieder aus der Aufnahme entfernt wird.

14. System aus einem Befüllroboter und einem Magazin (100) nach einem der Ansprüche 1 bis 11, wobei der Befüllroboter eine Aufnahme umfasst, in welcher das Magazin (100) passgenau aufgenommen werden kann.

## Claims

1. Magazine (100) having a plurality of solution bags (20) for dialysis received therein, wherein the plurality of solution bags (20) are identical, **characterized in that** the plurality of solution bags (20) are fixed in a stationary manner in an identical orientation at different, but identically designed holding positions of the magazine (100), and wherein the holding positions are configured such that the solution bags (20) can be removed from the magazine (100).

2. Magazine (100) in accordance with claim 1, **characterized in that** the solution bags (20) are bags made of connected plastic films.

3. Magazine (100) in accordance with one of the preceding claims, **characterized in that** the solution bags (20) have a material projection which is not in direct contact with the internal volume, wherein the material projection preferably is a peripheral weld seam (21).

4. Magazine (100) in accordance with one of the preceding claims, **characterized in that** the magazine (100) comprises a plurality of identical carriers (10) in which a respective one solution bag (20) is fixed, wherein the carriers (10) are stacked directly at one another.

5. Magazine (100) in accordance with claim 4, **characterized in that** the carriers (10) have an areal base element and a framework (12) extending in the normal direction, wherein the solution bag (20) lies on the base element and is surrounded by the framework (12).

6. Magazine (100) in accordance with claim 5, **characterized in that** the carriers (10) have at least one pin (13) that projects in the normal direction from the base element.

7. Magazine (100) in accordance with one of the claims 1 to 3, **characterized in that** the magazine (100) has a common frame (30) having a plurality of slots (33) at which the plurality of solution bags (20) are placed.

8. Magazine (100) in accordance with claim 7, **characterized in that** the frame (30) has a perforated plate (31) having a plurality of elongate holes and the solution bags (20) each have a peg, wherein the elongate holes each have a slot (33) at an end of the elongate hole and have an extended portion at the other end or are open toward the edge of the perforated plate, and wherein the pegs have a collar whose diameters are larger than the width of the elongate hole, but smaller than the diameter of the extended portion.

9. Magazine (100) in accordance with claim 7 or 8, **characterized in that** the frame (30) is releasably fastened to a box (40).

10. Magazine (100) in accordance with one of the preceding claims, **characterized in that** the solution bags (20) are filled with a concentrate for preparing a dialysis solution.

11. Magazine (100) in accordance with claim 10, **characterized in that** the concentrate takes up less than 40% of the volume capacity of the bag.

12. Method of filling the solution bags (20) of a magazine (100) in accordance with one of the preceding claims, wherein the magazine (100) is inserted into a filling robot and individual solutions bags (20) are then removed from the magazine (100) and then filled or are filled and then removed from the magazine (100) as part of a preferably automated process.

13. Method in accordance with claim 12, **characterized in that** the magazine (100) is automatically introduced into a receiver of the filling robot so that the solution bags (20) are present in a position suitable for the removal and, optionally, filling and the magazine (100) is automatically removed from the receiver again after the removal.

14. System of a filling robot and a magazine (100) in accordance with one of the claims 1 to 11, wherein the filling robot comprises a receiver in which the magazine (100) can be received in an exact fit.

## Revendications

1. Magasin (100) avec plusieurs sachets de solution (20) pour la dialyse logés à l'intérieur, les plusieurs sachets de solution (20) étant identiques, **caractérisé en ce que** les plusieurs sachets de solution (20) sont fixés de manière stationnaire à des emplacements de retenue différents mais de configuration identique du magasin (100) dans une orientation identique et les emplacements de retenue étant configurés de telle sorte que les sachets de solution (20) peuvent être retirés du magasin (100).

2. Magasin (100) selon la revendication 1, **caractérisé en ce que** les sachets de solution (20) consistent en des sachets de films plastiques reliés.

3. Magasin (100) selon l'une quelconque des revendications précédentes, **caractérisé en ce que** les sachets de solution (20) présentent un surplus de matière qui n'est pas en contact direct avec le volume intérieur, le surplus de matière consistant de préférence en un cordon de soudure périphérique (21).

4. Magasin (100) selon l'une quelconque des revendications précédentes, **caractérisé en ce que** le magasin (100) présente une pluralité de supports identiques (10) dans chacun desquels est fixé un sachet de solution (20), les supports (10) étant empilés directement les uns sur les autres.

5. Magasin (100) selon la revendication 4, **caractérisé en ce que** les supports (10) présentent un élément de base plat et un cadre (12) s'étendant dans la direction normale, le sachet de solution (20) reposant sur l'élément de base et étant entouré par le cadre (12).

6. Magasin (100) selon la revendication 5, **caractérisé en ce que** les supports (10) présentent au moins une tige (13) faisant saillie de l'élément de base dans la direction normale.

7. Magasin (100) selon l'une quelconque des revendications 1 à 3, **caractérisé en ce que** le magasin (100) présente un cadre commun (30) avec plusieurs emplacements d'enfichage (33), sur lesquels sont enfichés les plusieurs sachets de solution (20).

8. Magasin (100) selon la revendication 7, **caractérisé en ce que** le cadre (30) présente une plaque perforée (31) avec plusieurs trous oblongs et les sachets de solution (20) présentent chacun un tenon, les trous oblongs présentant chacun un emplacement d'enfichage (33) à une extrémité du trou oblong ainsi qu'un élargissement à l'autre extrémité ou étant ouverts vers le bord de la plaque perforée, et les tenons présentant une collerette dont le diamètre est supérieur à la largeur du trou oblong mais inférieur au diamètre de l'élargissement.

9. Magasin (100) selon la revendication 7 ou 8, **caractérisé en ce que** le cadre (30) est fixé de manière amovible à une boîte (40).

10. Magasin (100) selon l'une quelconque des revendications précédentes, **caractérisé en ce que** les sachets de solution (20) sont remplis d'un concentré pour la préparation d'une solution de dialyse.

11. Magasin (100) selon la revendication 10, **caractérisé en ce que** le concentré occupe moins de 40 % du volume du sachet.

12. Procédé de remplissage des sachets de solution (20) d'un magasin (100) selon l'une quelconque des revendications précédentes, le magasin (100) étant placé dans un robot de remplissage, puis des sachets de solution individuels (20) étant soit retirés du magasin (100) et ensuite remplis, soit remplis et ensuite retirés du magasin (100) dans le cadre d'un processus de préférence automatisé.

13. Procédé selon la revendication 12, **caractérisé en ce que** le magasin (100) est introduit automatiquement dans un logement du robot de remplissage, de telle sorte que les sachets de solution (20) se trouvent dans une position appropriée pour le retrait et éventuellement le remplissage, et le magasin (100) est sorti automatiquement du logement après le retrait.

14. Système composé d'un robot de remplissage et d'un magasin (100) selon l'une quelconque des revendications 1 à 11, le robot de remplissage comprenant un logement dans lequel le magasin (100) peut être logé de manière ajustée.
